# EUROPEAN PATENT APPLICATION

(11) **EP 4 804 198 A1**
(43) Date of publication of application: **09.09.2026**
(21) Application number: 26163204.6
(22) Date of filing: 09.03.2026
(51) Int. Cl.: G16H 10/60, G16H 15/00, G16H 70/20

(54) **SYSTEM AND METHOD FOR STANDARDIZING ELECTRONIC MEDICAL RECORDS**

(30) Priority: 07.03.2025 TW 114108606; 11.08.2025 TW 114130610
(71) Applicant: Ministry of Health and Welfare, Taipei City 115204 (TW)
(72) Inventor: LEE, Chien-Chang, 115204 Taipei City (TW)
(74) Representative: Michalski Hüttermann & Partner mbB

(57) **Abstract**

The present invention provides a system and method for standardizing electronic medical records. Non-standardized medical records from various sources are processed by a format conversion procedure to generate an unstructured electronic medical record, and may optionally undergo format validation. The unstructured electronic medical record is processed using algorithms comprising at least one large language model to segment and refine the text, extract at least one keyword set, and combine the keyword set with a code name set from at least one international medical code set to generate a plurality of query sets. The query sets are matched against medical terms to select a code name and code number combination associated with a highest confidence index, thereby forming at least one mapping entry. A GUI displays the unstructured text and the mapping entry to facilitate review, flagging, and modification, thereby generating a standardized electronic medical record.

## Description

### FIELD OF THE INVENTION

The present invention relates to a system for electronic medical records, and more particularly to a system for standardizing electronic medical records. The present invention further relates to a method associated with the electronic medical record standardization system.

### BACKGROUND OF THE INVENTION

Since 2000, Taiwan has actively promoted the development of electronic medical records. In 2004, a basic format for electronic medical record content (TMT) was established, and inter-hospital exchange of electronic medical records began in 2006. By 2014, 355 hospitals had passed electronic medical record inspections, and 343 hospitals had completed integration with the Electronic Medical Record Exchange Center (EEC). As of 2019, more than 90% of hospitals had implemented electronic medical record systems. However, existing electronic medical record systems still face issues such as system incompatibility, difficulty in patient referral, limitations in quality-indicator management, and a lack of a unified platform for smart healthcare applications. In particular, hospital information systems (HIS) at medical centers are highly customized, making data interoperability difficult and thereby affecting patient referral and quality of care.

To address these issues, Taiwan has been actively attempting and promoting adoption of the FHIR (Fast Healthcare Interoperability Resources) standard, which has been widely used internationally. The FHIR standard was developed by HL7 in 2011 and improves the efficiency of medical data exchange through a RESTful API architecture and modular design. Currently, the United States has incorporated FHIR into the 21st Century Cures Act; the National Health Service (NHS) of the United Kingdom, as well as countries such as Germany and Japan, have also adopted the FHIR standard, and some medical institutions in China are likewise attempting to align with international practices.

FHIR (Fast Healthcare Interoperability Resources) itself is merely a conversion tool. To truly achieve interoperability and circulation of medical data, further standardization operations are still required, including laboratory data standardization (LOINC), clinical record standardization (SNOMED CT), and medication prescription standardization (RxNorm). However, these standardization processes are highly complex and time-consuming. The primary challenges stem from differences in recording practices across hospitals. Even after conversion into the FHIR format, accurate alignment may remain difficult due to differences in names, units, and recording methods.

For example, LOINC standardization requires unified coding for all laboratory test items, which involves not only verification of large volumes of historical records, but also one-by-one comparison against international standards to ensure correctness of the conversion. Similarly, SNOMED CT standardization covers clinical records such as medical record summaries, diagnoses, and imaging reports. Because physicians may have different writing styles, semantic ambiguity can be significant, and manual classification and comparison by professionals is often required. In addition, RxNorm standardization is also highly challenging because national health insurance drug codes used by hospitals in Taiwan are not fully compatible with U.S. RxNorm, and some drugs may not even have directly corresponding standard codes, thereby further complicating the conversion process.

On the other hand, the large volume of historical data to be converted is itself a major difficulty. Medical institutions in Taiwan have accumulated substantial amounts of non-standardized medical records. To make such data compliant with the FHIR standard, legacy medical records need to be re-coded. However, older record formats may differ significantly from current standards; for example, diagnosis names may have been entered in handwriting and thus require manual mapping to SNOMED CT codes. Many physician notes, surgical records, or imaging reports are unstructured text, making standardization even more challenging.

To overcome these standardization difficulties, artificial intelligence (Al) technologies are becoming a key solution. Natural language processing (NLP) can automatically parse text and improve the degree of structuring and consistency of data through semantic analysis. Large language models may further enable automatic identification and classification into FHIR standard resources, such as Observation (test results), Condition (clinical conditions), and Medication Request (prescriptions), and may even attempt to fill in missing information.

However, AI technologies still carry a risk of misclassification. In particular, in Taiwan, physicians may write or enter medical record information in Chinese and English, or even a mixture of additional languages. Further, differences in writing styles among physicians, spelling errors, code-switching, and use of abbreviations may also affect the accuracy of standardization. For example, "DM" may refer to diabetes mellitus, or may refer to dermatomyositis; "HTN" may indicate hypertension, but in some literature it may also refer to an adrenal tumor (hypertonic neoplasm).

In addition, medication names may be recorded differently across hospitals. For example, "Lasix" (a diuretic) may be recorded only by its generic name "furosemide" in some medical records, while in other medical records it may be recorded by brand names such as "Advil" and "Motrin," which are products having the generic name "ibuprofen." When brand names and generic names are mixed without effective normalization and unification, mapping to standardized medication codes (e.g., RxNorm) becomes difficult, which may in turn lead to inconsistency in medical information and inaccuracies in data exchange. In particular, in cross-institution and cross-platform data integration or clinical decision support applications, such differences in medication naming may further affect the quality of automated interpretation and analysis. Accordingly, even though AI can substantially reduce manual burden, review and correction by medical and information professionals are still needed at the present stage to ensure accuracy and compliance of standardized data.

### SUMMARY OF THE INVENTION

The present invention provides a system and a method for standardizing electronic medical records. In addition to processing non-standardized medical records from different sources through a format conversion procedure (e.g., FHIR format conversion) and a format validation procedure (e.g., FHIR format validation) to generate an unstructured electronic medical record, the present invention further applies algorithms to assist in the conversion and standardization of electronic medical records, thereby improving interpretation accuracy and structuring quality for the unstructured electronic medical record. The present invention further provides a graphical user interface (GUI) to assist a user in quickly reviewing and modifying algorithmic results, thereby improving the efficiency of electronic medical record standardization.

As one principal embodiment among a plurality of embodiments, the present invention relates to a system for standardizing electronic medical records. The system comprises: (1) at least one storage device storing a non-standardized medical record, an unstructured electronic medical record, at least one international medical code set, and a standardized electronic medical record; (2) a computer comprising a processor coupled to the at least one storage device and configured to execute a FHIR format conversion procedure, a FHIR format validation procedure, and at least three algorithms, to process the non-standardized medical record into the unstructured electronic medical record through the format conversion procedure and the format validation procedure, to process the unstructured electronic medical record into an unstructured text through the at least three algorithms, to further process the unstructured text to generate at least one mapping entry, and to generate the standardized electronic medical record by mapping the at least one mapping entry to the unstructured text; and (3) an electronic device comprising: a display device configured to display a GUI that displays the unstructured text and the at least one mapping entry; and a processor couplable to the at least one storage device and the computer to receive information from the GUI, to generate the standardized electronic medical record, and to transmit the standardized electronic medical record to the at least one storage device.

In some embodiments, the non-standardized medical records from different sources first undergo a preprocessing step (Step 0), including processing a non-standardized medical record into an unstructured electronic medical record by a FHIR format conversion procedure, and optionally performing a FHIR format validation procedure to ensure that the contents comply with applicable standard specifications. Thereafter, steps for processing the unstructured electronic medical record by the at least three algorithms and generating a standardized electronic medical record comprise:
Step 1: uploading an unstructured electronic medical record from the at least one storage device to the computer, and executing, by the processor, a refinement algorithm, wherein at least one large language model performs readability preprocessing on the unstructured electronic medical record, including segmenting the unstructured electronic medical record into a multi-segment text, and performing translation, abbreviation expansion, grammar and spelling correction, semantic smoothing, and supplementation of details for the multi-segment text, thereby processing the multi-segment text into an unstructured text, such that different types of information can be independently analyzed and processed to avoid confusion;
Step 2: inputting the unstructured text into a structuring algorithm, wherein at least one large language model extracts at least one keyword set from at least one paragraph of the unstructured text according to the at least one international medical code set, extracts at least one code name set from the at least one international medical code set, and generates a plurality of query sets based on the keyword set(s), the code name set(s), and combinations thereof;
Step 3: inputting the plurality of query sets into a mapping algorithm to search and match the plurality of query sets with a plurality of medical terms in a medical database, wherein each medical term comprises a code combination composed of at least one code name and a corresponding code number; calculating a relevance score according to a number of times each medical term is successfully matched by the plurality of query sets, and selecting multiple top-k medical terms based on the relevance score; calculating, by at least one large language model, a confidence index based on semantic similarity between each medical term among the multiple top-k medical terms and the unstructured text; selecting at least one target term having a relatively high confidence index (e.g., the highest confidence index, a tied-highest confidence index, or a confidence index within a predefined range of the highest); forming at least one mapping entry using at least one code name and a corresponding code number in the code combination of the at least one target term; and generating a standardized electronic medical record by mapping the at least one mapping entry to the unstructured text.

In some preferred embodiments, the steps for generating a standardized electronic medical record further comprise Step 4: updating the confidence index by repeatedly performing Steps 1 to 3 using the at least one large language model, and by updating the confidence index through an algorithm. According to a number of large language models employed and a number of runs, the algorithm comprises: a single-model confidence index, selecting the highest confidence from a single model, averaging multiple runs of a single model, selecting the highest confidence from multiple models, averaging confidence from multiple models, or averaging multiple runs of multiple models; and forming the at least one mapping entry using at least one code name having the highest updated confidence index in the code combination and a corresponding code number, and generating a standardized electronic medical record by mapping the at least one mapping entry to the unstructured text.

In some more preferred embodiments, the steps for generating a standardized electronic medical record further comprise Step 5: displaying, by the GUI, the unstructured text and the at least one mapping entry, wherein each mapping entry is further displayed with a confirmation flag and a modification flag; and wherein the confirmation flag and the modification flag are displayed based on results calculated by inputting the confidence index into a flagging strategy, so as to assist a user in quickly confirming correctness of each mapping entry and generating the standardized electronic medical record after any necessary modification and supplementation.

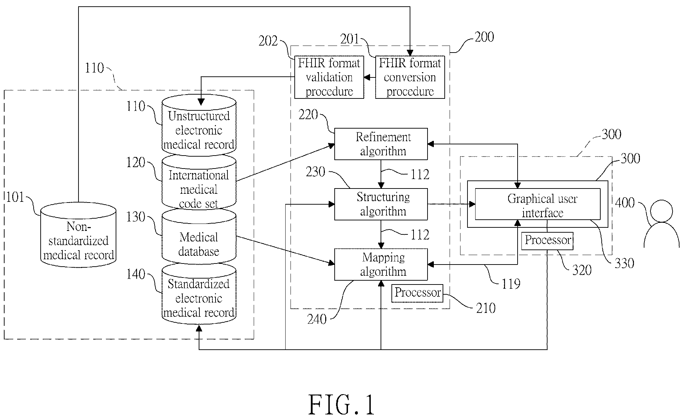

The foregoing and other embodiments are described in further detail below with reference to FIGS. 1-10. Although numerous examples are described in the context of system devices, electronic medical record conversion processing, algorithmic processing steps, and graphical user interfaces, the techniques described herein are applicable to other types of devices, functions, and applications.

### BRIEF DESCRIPTION OF THE DRAWINGS

FIG. 1 is a schematic diagram of an electronic medical record standardization system according to the present invention.
FIG. 2 is a flowchart illustrating a process for standardizing a non-standardized medical record in the electronic medical record standardization system according to the present invention.
FIG. 3 is a schematic diagram illustrating a refinement algorithm of the electronic medical record standardization system according to the present invention.
FIG. 4 is a schematic diagram illustrating a structuring algorithm of the electronic medical record standardization system according to the present invention.
FIG. 5 is a schematic diagram illustrating a mapping algorithm of the electronic medical record standardization system according to the present invention.
FIG. 6 is a schematic diagram illustrating a graphical user interface (GUI) of the electronic medical record standardization system according to the present invention.
FIG. 7 is a schematic diagram illustrating a system-flag function of the graphical user interface of the electronic medical record standardization system according to the present invention.
FIG. 8A is a schematic diagram illustrating a modification function of the graphical user interface of the electronic medical record standardization system according to the present invention.
FIG. 8B is a schematic diagram illustrating a supplementation function of the graphical user interface of the electronic medical record standardization system according to the present invention.
FIG. 9 is a schematic diagram illustrating a focus flag function of the graphical user interface of the electronic medical record standardization system according to the present invention.
FIG. 10 is a schematic diagram illustrating a refinement toggle of the graphical user interface of the electronic medical record standardization system according to the present invention.

### DETAILED DESCRIPTION OF THE INVENTION

In the following description, numerous specific embodiments are set forth to provide a thorough understanding of the present invention. However, persons having ordinary skill in the art will appreciate that the present invention may be practiced without some or all of these specific details, without departing from the scope of the present invention. In addition, to avoid unnecessarily obscuring the present invention, certain well-known techniques, methods, or computational procedures are not described in detail herein, although such techniques, methods, or procedures will be apparent to those skilled in the art.

The following detailed description refers to the accompanying drawings, which form a part of this specification and illustrate embodiments of the present invention. These embodiments (also referred to herein as "examples") are described in sufficient detail to enable those skilled in the art to practice the present invention. However, various embodiments may be combined, substituted, or modified in structure, logic, and processing manner without departing from the scope of the present invention as claimed. Accordingly, the following detailed description is not to be construed as limiting the present invention, and the scope of the present invention is defined by the appended claims and their equivalents.

As used herein, unless expressly specified otherwise, the terms "a" and "an" are to be understood as including "at least one." Further, the term "or" is to be interpreted as non-exclusive, such that "A or B" includes "A but not B," "B but not A," and "both A and B," unless the context clearly indicates otherwise.

The present invention relates to a system and a method for standardizing non-standardized medical records. Non-standardized medical records may originate from different sources and, after being processed by a format conversion procedure (e.g., FHIR format conversion) and a format validation procedure (e.g., FHIR format validation), are converted into an unstructured electronic medical record. By applying algorithms that include at least one large language model (LLM), the unstructured electronic medical record can be processed by refinement, structuring, and mapping, thereby generating a standardized electronic medical record compliant with applicable standards. The present invention not only improves readability and structuring quality of medical records, but also provides a graphical user interface (GUI) to enable a user to quickly review, modify, and confirm the results, thereby improving the efficiency of electronic medical record standardization.

In some embodiments, the system and method of the present invention comprise: at least one storage device configured to store a non-standardized medical record, an unstructured electronic medical record, and a standardized electronic medical record; a computer comprising a processor and configured to execute a FHIR format conversion procedure, a FHIR format validation procedure, and at least three algorithms, to process the non-standardized medical record into the unstructured electronic medical record, to further convert the unstructured electronic medical record into an unstructured text, and to further perform mapping with at least one international medical code set (e.g., ICD-10, SNOMED CT, and LOINC) to generate the standardized electronic medical record; and an electronic device comprising: a display device configured to display a graphical user interface to present the unstructured text and at least one mapping entry; and a processor couplable to the at least one storage device and the computer, so as to generate and store the standardized electronic medical record.

Compared with conventional electronic medical record standardization techniques, the present invention has the following technical advantages: (1) Automated and efficient processing: conventional standardization relies on manual coding and review, which is cumbersome and prone to error. The present invention, by using algorithms including at least one large language model to automatically extract key information, refine contents, and perform mapping with international medical code sets, can significantly reduce manual workload and improve standardization efficiency. (2) Improved accuracy and consistency: before algorithmic processing, non-standardized medical records from different sources are first processed by a FHIR format conversion procedure, such that original record contents are preliminarily aligned to a standardized field schema, thereby forming an unstructured electronic medical record with consistent field structures. In addition, the unstructured electronic medical record may optionally undergo a FHIR format validation procedure to ensure that field contents comply with the FHIR standard and an implementation guide, thereby avoiding issues such as missing data, format errors, and invalid codes. This processing order effectively improves subsequent accuracy and processing efficiency of the language model in semantic recognition, field parsing, and terminology standardization. Since records after FHIR format conversion have been categorized according to structured fields (e.g., "chief complaint," "observation," and "procedure"), the language model can analyze contents of each field in a more targeted manner to reduce contextual confusion risks. Meanwhile, executing the FHIR format validation procedure can eliminate formatting errors such as missing or misplaced fields, thereby avoiding misleading subsequent inference and mapping computation, improving overall accuracy and output quality of standardized data, and facilitating integration with external systems (e.g., a FHIR gateway and an EHR platform). Thereafter, through a refinement algorithm, the system can perform text segmentation, unified translation, abbreviation expansion, grammar and spelling correction, drug name conversion, semantic adjustment, and supplementation of details to ensure high readability of the unstructured text. Through a structuring algorithm, the system can extract keywords and code names based on contextual analysis and international medical code sets, further combine them into a plurality of query sets to match medical terms in a medical database, and filter results based on a relevance score and a confidence index, thereby further improving mapping accuracy with international medical code sets. (3) Interactive review and correction: a graphical user interface is provided to display mapping entries generated by the algorithm, enabling a user to quickly confirm and modify the results. The graphical user interface may display code names, code numbers, keywords, a confidence index, and a flagging strategy to help the user quickly inspect mapping results and make necessary adjustments. (4) Self-learning and continuous optimization: the system and method of the present invention can repeatedly train and continuously update algorithm models based on the standardized electronic medical record corrected and confirmed by the user, thereby improving mapping accuracy.

To achieve the foregoing technical objectives, the method of the present invention mainly comprises the following steps.

Step 0 (Preprocessing). Processing a non-standardized medical record into an unstructured electronic medical record by a FHIR format conversion procedure, and optionally performing a FHIR format validation procedure to ensure that the contents comply with standard specifications.

Step 1 (Refinement). Performing readability preprocessing on the unstructured electronic medical record by a refinement algorithm, such as text segmentation, translation, abbreviation expansion, grammar and spelling correction, semantic smoothing, and supplementation of details, to generate an unstructured text.

Step 2 (Structuring). Extracting at least one keyword set and at least one code name set from at least one paragraph of the unstructured text according to at least one international medical code set by a structuring algorithm, and generating a plurality of query sets by combining the extracted sets.

Step 3 (Mapping). Matching the plurality of query sets with a plurality of medical terms in a medical database, and forming at least one mapping entry using at least one code name and a code number included in at least one target term having a relatively high confidence index.

Step 4 (User Review). Displaying the unstructured text and the at least one mapping entry via a graphical user interface to allow a user to quickly review, flag, and correct the results to produce a standardized electronic medical record.

In some embodiments, the graphical user interface may display at least one flagging strategy. The flagging strategy may set a threshold based on the confidence index to automatically display a confirmation flag and a modification flag, thereby prompting whether further review and modification of the at least one mapping entry is needed. In addition, the graphical user interface may support a supplementation flag and a supplementation field, allowing a user to add notes for specific contents to improve completeness and accuracy of the standardized electronic medical record.

In some preferred embodiments, the standardized electronic medical record may further serve as training data and be fed back into the structuring algorithm and the mapping algorithm for model optimization, thereby improving accuracy of keyword extraction and mapping through iterative learning.

In some embodiments, the storage device may be any device or system capable of storing electronic medical records, including, but not limited to, an on-premises storage device, a cloud storage device, or a distributed storage architecture. For example, the storage device may comprise: a non-volatile storage device, such as a solid-state drive (SSD), a hard disk drive (HDD), flash memory, or other local storage media; a server or database system, such as an electronic health record (EHR) database deployed within a medical institution; or cloud storage resources in a remote server, a private cloud, a public cloud, or a hybrid cloud environment.

In various embodiments, the storage device may be coupled to the computer and/or the electronic device in various forms, including, but not limited to: wired coupling, such as via transmission protocols including PCIe, SATA, USB, fiber optics, or Ethernet; and wireless communication, such as via Wi-Fi, Bluetooth, near-field communication (NFC), Zigbee, a cellular network (e.g., 4G or 5G), or other wireless transmission technologies for data access and exchange.

In some embodiments, the storage device may be a standalone device, such as a server or database system deployed within a medical institution, and may exchange data with other systems via a local area network (LAN) or a wide area network (WAN). In other embodiments, the storage device may be a component of the computer or the electronic device, for example, as internal storage built into the computer or the electronic device, or as a removable storage device such as a USB storage device or an SD memory card.

In some preferred embodiments, the storage device may be implemented using a single storage architecture or a combination of multiple storage architectures, so as to provide scalability, security, and access efficiency. For example, some applications may combine on-premises storage with cloud storage, such that a portion of data is stored locally to improve access speed, while other data is stored in the cloud to facilitate remote access and backup management.

In some embodiments, the electronic device is used to display a graphical user interface (GUI), so that a user can review, operate, and confirm standardized contents of an electronic medical record. The electronic device may be any device having a display device and a processor, including, but not limited to: a personal computer (PC), a laptop computer, or a workstation; a mobile device such as a smartphone or a tablet computer; or other electronic devices with display capability, such as a medical-dedicated terminal, a touch display, or an embedded system.

In some embodiments, the electronic device may operate independently (i.e., as a device different from the computer) and may be connected to the computer through wired communications (e.g., USB, HDMI, or Ethernet) or wireless communications (e.g., Wi-Fi, Bluetooth, 5G, or NFC), to obtain the unstructured text and the at least one mapping entry generated by algorithmic processing and to allow the user to review and confirm. The processor of the electronic device may further process user inputs and transmit the confirmed standardized electronic medical record back to the computer for subsequent storage and application.

In another embodiment, the electronic device and the computer may be the same device, in which the computer includes a built-in display device and a processor and executes the graphical user interface, so that the user can directly review and operate on the computer. In this scenario, the processor of the computer is responsible for performing algorithmic processing, displaying the graphical user interface, and generating the standardized electronic medical record based on the user's confirmation results, without requiring an additional standalone electronic device.

In some preferred embodiments, the electronic device may support different operation modes according to usage needs. For example, in some application scenarios, the user may perform preliminary review and edits on a mobile device (such as a tablet or a smartphone) and then synchronize final results to the computer for further processing and storage. In other application scenarios, the user may complete all steps directly via the display device and the processor of the computer, thereby simplifying the operational workflow.

In some embodiments, the algorithms include at least one large language model (LLM) to perform refinement, structuring, and mapping on the unstructured electronic medical record. The large language model has natural language processing (NLP) capabilities and can understand, analyze, transform, and generate medical texts, so that the unstructured electronic medical record can be processed to satisfy standardization requirements.

However, the present invention is not limited to using a specific large language model. Any model having core capabilities associated with large language models, for example: language understanding and semantic parsing capable of grammar correction, contextual reasoning, and semantic analysis; text generation and refinement capable of refining content, unifying terminology, expanding abbreviations, and improving readability; text structuring capable of converting unstructured text into a standardized format; and keyword extraction and entity linking capable of recognizing medical terms and mapping them to at least one international medical code set (e.g., ICD-10, SNOMED CT, and LOINC), may be applied to the present invention. For example, in addition to deep learning-based large language models, the present invention is also applicable to: small language models; specialized medical language models (e.g., BioBERT, ClinicalBERT, and Med-PaLM); hybrid models combining rule-based NLP and statistical language models; or other machine learning or knowledge-graph-based approaches that provide large-language-model-like functionality while adopting different architectures.

In some preferred embodiments, the algorithms may be adapted according to different application scenarios. For example, in an environment rich in medical professional terminology, a large language model trained on large-scale medical datasets or a dedicated medical NLP model may be adopted to improve semantic understanding and standardization accuracy; in resource-constrained environments or environments with high privacy requirements, an on-premises deployment variant of a large language model may be used to provide data security and computational efficiency; and in applications with stringent standardization requirements, multiple types of algorithms may be integrated, such as a large language model together with rule-based methods or knowledge-base query methods, to improve accuracy of standardized medical records. Accordingly, the technical solutions of the present invention are applicable to current and future large language model technologies and are not limited to a specific language model architecture. Any algorithm or machine learning method that provides the above large language model advantages may be used to implement the present invention to improve standardization quality of electronic medical records.

In some embodiments, the graphical user interface and the standardized electronic medical record may further be applied to various clinical and statistical analysis scenarios, including patient disease-course tracking, regional healthcare utilization statistics, disease trend monitoring, and medical institution service evaluation, where the graphical user interface provides intuitive statistical data support.

The following description will further illustrate detailed structures and operations of the present invention with reference to specific embodiments and the accompanying drawings.

FIG. 1 illustrates a schematic diagram of an electronic medical record standardization system according to the present invention. As shown in FIG. 1, in some embodiments, the system includes at least one storage device 100, a computer 200, and an electronic device 300. The storage device 100 includes a non-standardized medical record 101, an unstructured electronic medical record 110, at least one international medical code set 120, a medical database 130, and a standardized electronic medical record 140. The computer 200 includes a processor 210 coupled to the at least one storage device 100 and is configured to execute a FHIR format conversion procedure 201 and a FHIR format validation procedure 202 to process the non-standardized medical record 101 into the unstructured electronic medical record 110. The computer 200 may further execute at least three algorithms, including a refinement algorithm 220, a structuring algorithm 230, and a mapping algorithm 240, to process the unstructured electronic medical record 110 into an unstructured text 112 and to generate at least one mapping entry 119. The electronic device 300 includes a display device 310 and a processor 320. The display device 310 is configured to display a graphical user interface 330 that displays at least the unstructured text 112 and the at least one mapping entry 119. The processor 320 is couplable to the at least one storage device 100 and the computer 200 and receives information from the graphical user interface 330 to generate the standardized electronic medical record 140 and to transmit the standardized electronic medical record 140 to the at least one storage device 100.

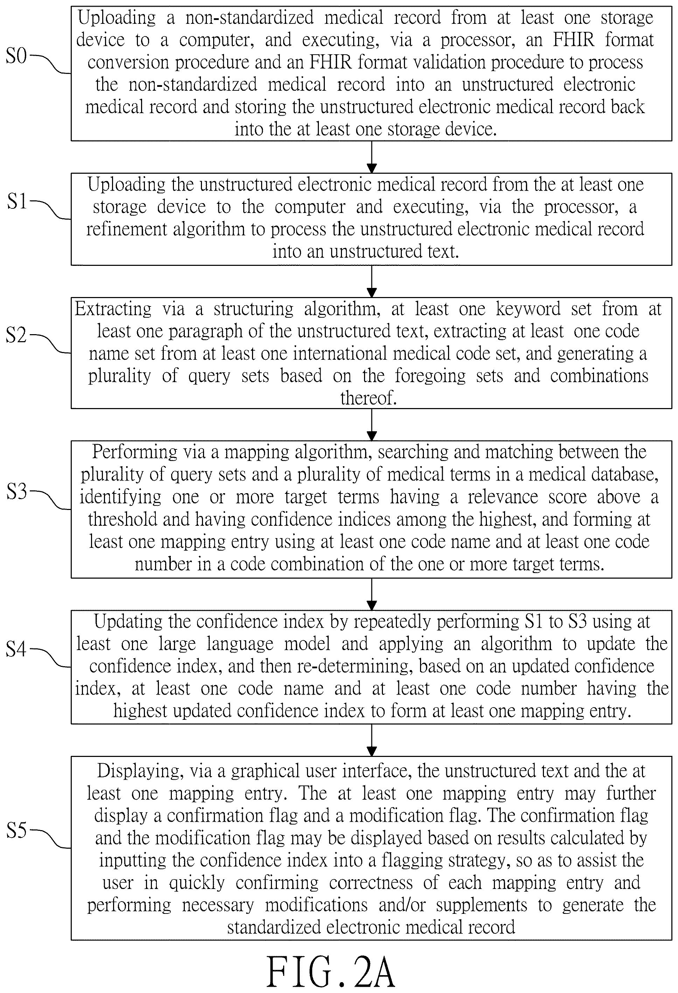

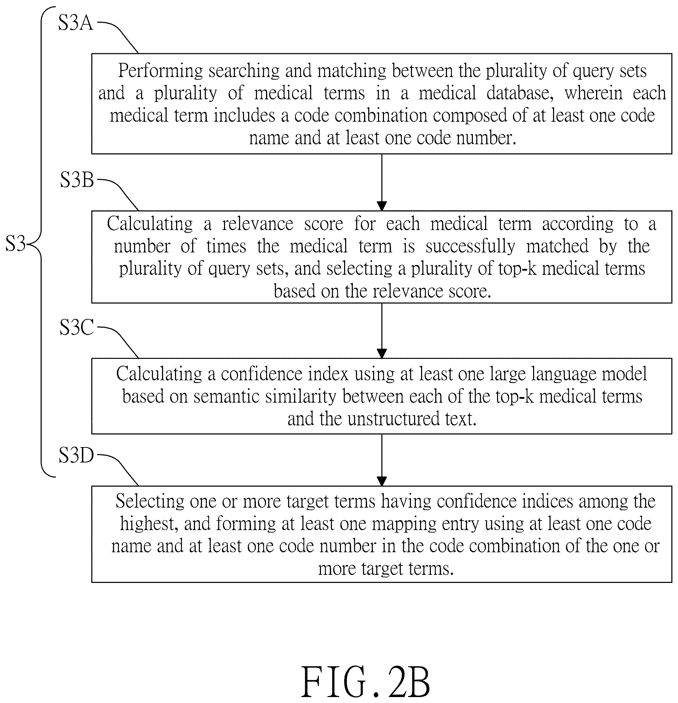

FIG. 2 illustrates a flowchart for standardizing a non-standardized medical record in the electronic medical record standardization system of the present invention. As shown in FIG. 2, in some embodiments, steps for processing a non-standardized medical record 101 using an FHIR format conversion procedure 201 and an FHIR format validation procedure 202, and further processing the resulting record using at least three algorithms to generate a standardized electronic medical record 140, include:
S0: uploading a non-standardized medical record 101 from at least one storage device 100 to a computer 200, and executing, via a processor 210, an FHIR format conversion procedure 201 and an FHIR format validation procedure 202 to process the non-standardized medical record 101 into an unstructured electronic medical record 110, and storing the unstructured electronic medical record 110 back into the at least one storage device 100;
S1: uploading the unstructured electronic medical record 110 from the at least one storage device 100 to the computer 200 and executing, via the processor 210, a refinement algorithm 220 to process the unstructured electronic medical record 110 into an unstructured text 112;
S2: extracting, via a structuring algorithm 230, at least one keyword set 114 from at least one paragraph 113 of the unstructured text 112, extracting at least one code name set 121 from at least one international medical code set 120, and generating a plurality of query sets 115 based on the foregoing sets and combinations thereof; and
S3: performing, via a mapping algorithm 240, searching and matching between the plurality of query sets 115 and a plurality of medical terms 131 in a medical database 130, identifying one or more target terms 117 having a relevance score 1161 above a threshold and having confidence indices 1191 among the highest, and forming at least one mapping entry 119 using at least one code name 121 and at least one code number 122 in a code combination 118 of the one or more target terms 117.

Thereby, the standardized electronic medical record 140 can be generated based on the at least one mapping entry 119 and the unstructured text 112.

In some preferred embodiments, the steps for generating the standardized electronic medical record 140 further include S4: updating the confidence index 1191 by repeatedly performing S1 to S3 using at least one large language model and applying an algorithm to update the confidence index 1191. This is intended to further improve accuracy by running the large language model multiple times and/or using different large language models (e.g., GPT-4, Claude, and Gemini) to obtain different results and comparing their outputs. If multiple large language models output consistent mapping entries 119, such mapping entries 119 may be assigned higher confidence indices 1191. If different large language models output different mapping entries 119, a final selection may be determined by voting and/or weighting, and the confidence index 1191 may be calculated and updated according to one or more algorithms, thereby making the decision process more objective and reliable. The algorithms may include:
Single Model Confidence, in which each large language model provides a confidence index 1191 associated with a mapping entry 119 output by that model (e.g., a model may return "SNOMED CT 25064002 (Headache)" with a confidence index 1191 of 90%, indicating a high match between the mapping entry 119 and the unstructured text 112);
Single Model Top Confidence, in which, if a single large language model produces multiple candidate mapping entries 119, a candidate having the highest confidence index 1191 is selected (e.g., a model may assign 90% to "25064002 (Headache)" and 70% to "37796009 (Migraine)," and thus "25064002" is selected);
Single Model Multi-Run Average, in which the same large language model is executed multiple times at different times and/or with different parameter settings and the confidence indices 1191 are averaged to reduce randomness (e.g., if three runs yield 85%, 90%, and 88%, the resulting confidence index 1191 is (85+90+88)/3 = 87.67%);
Multi-Model Top Confidence, in which, when multiple large language models each output at least one mapping entry 119 and a confidence index 1191, the mapping entry having the highest confidence index 1191 is selected (e.g., one model outputs Headache (0.90), another outputs Migraine (0.85), and another outputs Tension Headache (0.75), and thus the Headache result is selected);
Multi-Model Average Confidence, in which, if different large language models output the same code name 121, their confidence indices 1191 are averaged (e.g., two models both select Headache (25064002) with confidence indices 90% and 85%, respectively, and thus the resulting confidence index 1191 is (90+85)/2 = 87.5%); or
Multi-Model Multi-Run Average, in which multiple large language models are each executed multiple times, an average confidence index 1191 is obtained for each model, and the averages across models are further averaged to obtain a resulting confidence index 1191 (e.g., one model averages 87% across three runs, another averages 85%, and another averages 80%, and thus the resulting confidence index 1191 is (87+85+80)/3 = 84%).

In some more preferred embodiments, the steps for generating the standardized electronic medical record 140 further include S5: displaying, via a graphical user interface 330, the unstructured text 112 and the at least one mapping entry 119. The at least one mapping entry 119 may further display a confirmation flag 1192 and a modification flag 1193. The confirmation flag 1192 and the modification flag 1193 may be displayed based on results calculated by inputting the confidence index 1191 into a flagging strategy 3321, so as to assist the user in quickly confirming correctness of each mapping entry 119 and performing necessary modifications and/or supplements to generate the standardized electronic medical record 140.

FIG. 2 further shows that secondary steps of the mapping algorithm 240 in S3 include:
S3A: performing searching and matching between the plurality of query sets 115 and a plurality of medical terms 131 in a medical database 130, wherein each medical term 131 includes a code combination 118 composed of at least one code name 121 and at least one code number 122;
S3B: calculating a relevance score 1161 for each medical term 131 according to a number of times the medical term 131 is successfully matched by the plurality of query sets 115, and selecting a plurality of top-k medical terms 116 based on the relevance score 1161;
S3C: calculating a confidence index 1191 using at least one large language model based on semantic similarity between each of the top-k medical terms 116 and the unstructured text 112; and
S3D: selecting one or more target terms 117 having confidence indices 1191 among the highest, and forming at least one mapping entry 119 using at least one code name 121 and at least one code number 122 in the code combination 118 of the one or more target terms 117.

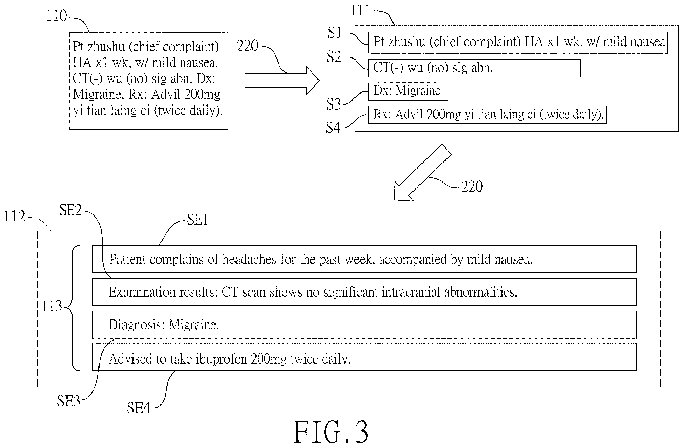

The refinement algorithm 220 of the present invention uses at least one large language model and is intended to improve readability of the unstructured electronic medical record 110. To illustrate processing of the refinement algorithm 220, FIG. 3 provides a specific embodiment showing how the refinement algorithm 220 processes the unstructured electronic medical record 110 into the unstructured text 112 through semantic analysis, content classification, and information segmentation. FIG. 3 illustrates a schematic diagram of the refinement algorithm 220 of the electronic medical record standardization system of the present invention.

The example text in FIG. 3, "Pt zhushu (chief complaint) HA x1 wk, w/ mild nausea. CT(-) wu (no) sig abn. Dx: Migraine. Rx: Advil 200mg yi tian laing ci (twice daily)." is incomplete, has low readability, and lacks standardized paragraph structures and sentence logic, which may cause semantic confusion during subsequent algorithmic processing. Therefore, the refinement algorithm 220 performs sentence recognition and segmentation to generate a multi-segment text 111, for example: "s1: Pt **zhushu** (chief complaint) HA x1 wk, w/ mild nausea.", "s2: CT(-) wu (no) sig abn.", "s3: Dx: Migraine.", and "s4: Rx: Advil 200mg yi **tian laing** ci (twice daily)."

Relying on contextual understanding and standardized transformation capabilities of the large language model, the refinement algorithm 220 may perform: content classification, which identifies different types of information in the text (e.g., chief complaint, examination results, diagnosis, and prescription) and assigns labels thereto so that different types of information can be processed separately; sentence segmentation, which divides the text into independent sentences based on semantic structure and punctuation rules to ensure clear hierarchical relationships among different types of information; and drug name normalization, which unifies representations of drug names appearing in the medical record. Drug name normalization may be performed using a pre-established mapping dictionary to convert a recognized brand name or product name into a corresponding generic name, for example, converting "Advil" or "Motrin" to "Ibuprofen," thereby improving consistency and accuracy of drug information in semantic recognition and standard-code mapping (e.g., RxNorm).

The refinement algorithm 220 then performs language correction, abbreviation expansion, grammar optimization, and semantic enhancement on the multi-segment text 111 to ensure that the resulting unstructured text 112 is easier to understand and analyze. In FIG. 3, the example text segments "s1: **Pt zhushu** (chief complaint) HA x1 wk, w/ mild nausea.", "s2: CT(-) wu (no) sig abn.", "s3: Dx: Migraine.", and "s4: Rx: Advil 200mg **yi tian laing** ci (twice daily)." are typical medical abbreviations and shorthand notes. Because such notes lack standard grammar and complete semantics, and because drug names are written using brand names, accuracy of automated processing and/or mapping may be reduced. Accordingly, the refinement algorithm 220 processes the foregoing segments to generate the unstructured text 112 with improved readability, for example: "se1: Patient complains of headaches for the past week, accompanied by mild nausea.", "se2: Examination results: CT scan shows no significant intracranial abnormalities.", "se3: Diagnosis: Migraine.", and "se4: Advised to take ibuprofen 200 mg twice daily."

Because large language models have capabilities in contextual understanding, text generation, and standardization, they can perform: abbreviation expansion to automatically convert professional abbreviations into full terms based on context, for example, converting "Pt" to "Patient," "HA" to "Headache," "w/" to "with," and "Rx" to "Prescription," thereby improving accuracy of abbreviation conversion; grammar correction and standardization to automatically correct grammar and unify formatting according to medical documentation standards, for example, converting "CT(-)**wu** (no) sig abn." to "CT scan shows no significant intracranial abnormalities."; terminology normalization to recognize and convert medical terms and drug names based on medical knowledge bases and drug dictionaries, for example, converting "Dx: Migraine" to "Diagnosis: Migraine." and converting "Rx: Advil 200mg **yi tian laing ci** (twice daily)" to "Advised to take ibuprofen 200 mg twice daily."; and semantic enhancement to analyze textual context, supplement omitted information, and reasonably expand expressions based on medical knowledge. For example, where "CT(-)" does not explicitly describe examination results, the large language model may produce a complete sentence such as "CT scan shows no significant intracranial abnormalities." to improve completeness of diagnostic information. Through processing by the refinement algorithm 220, subsequent structuring and mapping can be performed more accurately.

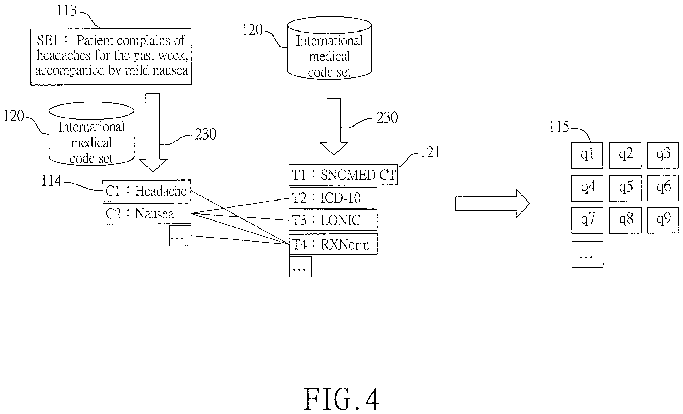

The structuring algorithm 230 of the present invention uses at least one large language model and is intended to analyze, summarize, and extract at least one keyword set 114 and at least one code name set 121 from the unstructured text 112 and at least one international medical code set 120, and then generate a plurality of query sets 115 based on the two sets and combinations thereof to provide reasonable and accurate references for subsequent mapping. To illustrate processing of the structuring algorithm 230, FIG. 4 provides a schematic diagram of the structuring algorithm 230 of the electronic medical record standardization system of the present invention.

The structuring algorithm 230 first processes at least one paragraph 113 after being processed by the refinement algorithm 220, for example, "se1: Patient complains of headaches for the past week, accompanied by mild nausea." Based on at least one international medical code set 120, the structuring algorithm 230 extracts the at least one keyword set 114 (C1, C2, ...) from the at least one paragraph 113 and extracts a code name set 121 (T1, T2, T3, T4, ...) from the at least one international medical code set 120. For example, the keyword set 114 may include (C1: "Headache," C2: "Nausea," C3: "Migraine," ...), and the code name set 121 may include (T1: "Pain in head," T2: "Vomiting sensation," T3: "Headache disorder," ...). Each item in the at least one keyword set 114 and the code name set 121, as well as combinations of the two sets, are then formed into the plurality of query sets 115 (q1, q2, q3, ...), for example, q1: "Headache," q2: "Nausea," q3: "Migraine," q4: "Pain in head," q5: "Vomiting sensation," q6: "Headache disorder," q7: ("Headache", "Pain in head"), q8: ("Nausea", "Vomiting sensation"), q9: ("Migraine", "Headache disorder"), and so on.

Also relying on the large language model, the structuring algorithm 230 employs a bidirectional matching mechanism to extract the at least one keyword set 114 and the at least one code name set 121 from both the unstructured text 112 and the at least one international medical code set 120 and to combine them to generate the plurality of query sets 115, so as to reduce incorrect mapping that may result from relying solely on keyword matching in the text. In addition, the plurality of query sets 115 may further include query sets 115 generated independently from the at least one keyword set 114 and query sets 115 generated independently from the at least one code name set 121, thereby expanding a query scope and improving completeness of mapping. Compared with conventional keyword-matching approaches, the present invention can adapt to semantic expressions of different medical standards and reduce matching errors caused by variations in medical terminology.

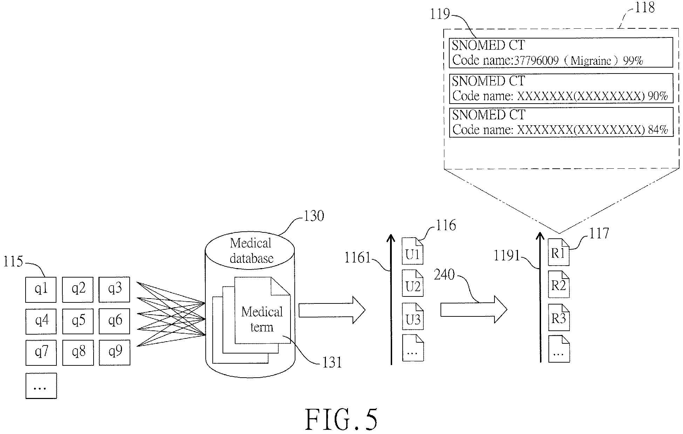

The mapping algorithm 240 of the present invention is intended to obtain at least one correct and accurate mapping entry 119. To illustrate processing of the mapping algorithm 240, FIG. 5 provides a schematic diagram of the mapping algorithm 240 of the electronic medical record standardization system of the present invention.

During processing by the mapping algorithm 240, the plurality of query sets 115 are input to a medical database 130 (e.g., UMLS, OMOP, and MedDRA) and matched against a plurality of medical terms 131 in the medical database 130. Each medical term 131 includes a code combination 118 composed of code names 121 and code numbers 122 from the at least one international medical code set 120. For example, "Migraine" may include SNOMED CT Migraine: 37796009, ICD-10-Migraine without aura: G43.0, and LOINC-Headache assessment: 92194-2; "Tension Headache" may include SNOMED CT-Tension-type headache: 25064002 and ICD-10-Tension-type headache: G44.2; and "Nausea" may include SNOMED CT-Nausea: 422587007 and ICD-10-Nausea: R11.0.

Based on matching results, a number of times each medical term 131 is successfully matched by the plurality of query sets 115 is counted, and a relevance score 1161 is calculated accordingly. For example, "Migraine" is successfully matched by q3, q6, and q9, and thus has a relevance score 1161 of 3; "Tension Headache" is successfully matched by q1, q4, and q7, and thus has a relevance score 1161 of 3; and "Nausea" is successfully matched by q2, q5, and q8, and thus has a relevance score 1161 of 3.

A plurality of top-k medical terms 116 (U1, U2, U3, ...) are selected based on the relevance scores 1161 of the plurality of medical terms 131, for example, selecting top 10 medical terms (U1, U2, U3, ... U10) according to ranking by relevance score 1161. A large language model then analyzes semantic matching between, for each of the top-k medical terms 116, the code combination 118 included in the medical term 131 and the unstructured text 112, and calculates a confidence index 1191 for each medical term accordingly, for example, "Migraine" SNOMED CT-Migraine: 37796009 (confidence index 0.92), "Tension Headache" SNOMED CT-Tension-type headache: 25064002 (confidence index 0.85), and "Nausea" SNOMED CT-Nausea: 422587007 (confidence index 0.80).

Finally, one or more target terms 117 having confidence indices 1191 among the highest are selected according to ranking by confidence index 1191, and at least one mapping entry 119 is formed using at least one code name 121 and at least one code number 122 in the code combination 118 of the one or more target terms 117, for example: R1: SNOMED CT-Migraine: 37796009 (Migraine); R2: SNOMED CT-Tension-type headache: 25064002 (Tension Headache); and R3: SNOMED CT-Nausea: 422587007 (Nausea).

The mapping algorithm 240 of the present invention ensures that results of electronic medical record standardization are accurate and explainable through large-language-model driven query matching, relevance-based filtering, contextual analysis, and confidence index evaluation. To improve reliability of matching, a statistical filtering mechanism is introduced, in which a number of times each medical term 131 is successfully matched by the plurality of query sets 115 is counted to select a plurality of top-k medical terms 116, thereby ensuring that subjects for subsequent contextual analysis are highly relevant, excluding low-relevance matches, and reducing a probability of incorrect mapping.

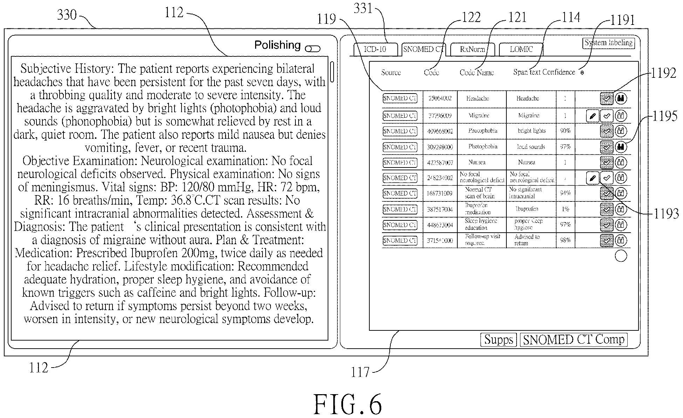

FIG. 6 illustrates a schematic diagram of the graphical user interface of the electronic medical record standardization system of the present invention. In addition to displaying the unstructured text 112 and the at least one mapping entry 119, the graphical user interface 330 further displays, for each mapping entry 119, a confirmation flag 1192, a modification flag 1193, at least one code name 121, at least one code number 122, and at least one keyword 114, allowing the user to further review and confirm correctness of each mapping entry 119. In some preferred embodiments, the confirmation flag 1192 and the modification flag 1193 are displayed based on results calculated by inputting the confidence index 1191 into a flagging strategy 3321, so as to assist the user in quickly confirming correctness of the at least one mapping entry 119 and performing necessary modifications and/or supplements. In some more preferred embodiments, the graphical user interface 330 further displays at least one tab 331 to summarize and display the at least one mapping entry 119 from different international medical code sets 120. In some embodiments, the at least one mapping entry 119 further displays an insurance flag 1195, allowing the user to manually mark whether the at least one mapping entry 119 falls within a health insurance reimbursement scope.

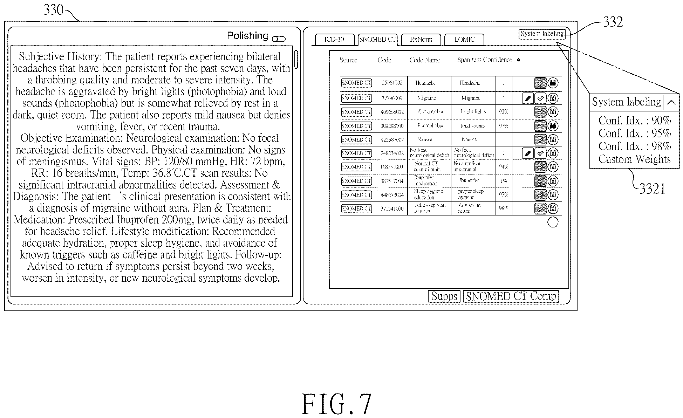

FIG. 7 illustrates a schematic diagram of a system-flag function of the graphical user interface of the electronic medical record standardization system of the present invention. The graphical user interface 330 further displays a system-flag field 332, which includes at least one flagging strategy 3321. Each flagging strategy 3321 may include a threshold that is used to determine whether the confidence index 1191 of each mapping entry 119 satisfies the threshold, and to display a corresponding confirmation flag 1192 and a modification flag 1193 based on the determination result, thereby enabling a user to quickly decide whether further review and modification of the mapping entry 119 is warranted.

For example, in a flagging strategy 3321 of "confidence index 95%," "95%" serves as a threshold for evaluating the confidence index 1191 of a mapping entry 119. When the confidence index 1191 exceeds the 95% threshold, the confirmation flag 1192 is displayed and the modification flag 1193 is not displayed. Conversely, when the confidence index 1191 is below the 95% threshold, the modification flag 1193 is displayed and the confirmation flag 1192 is not displayed.

In some more preferred embodiments, at least one flagging strategy 3321 may include customized weighting settings depending on the content and context of at least one paragraph 113 and a type of the corresponding international medical code set 120. For example, when the unstructured text 112 (and one or more paragraphs 113 thereof) primarily describes medication-related information, the code name 121 of a mapping entry 119 is, in principle, more relevant to a drug-related value set (e.g., RxNorm). In such a case, a user may increase a weight assigned to RxNorm in the customized weighting settings, such that mapping entries 119 associated with RxNorm are more likely to be selected and/or assigned higher confidence indices 1191, thereby making the confirmation flag 1192 more likely to be displayed.

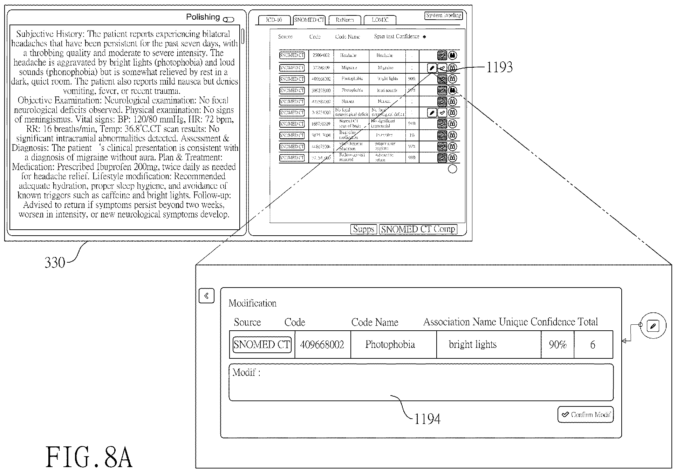

FIG. 8A illustrates a schematic diagram of a modification function of the graphical user interface of the electronic medical record standardization system of the present invention. In some embodiments, in addition to allowing a user to quickly confirm each mapping entry 119 via the confirmation flag 1192 displayed on the graphical user interface 330, when the confirmation flag 1192 is not displayed and the modification flag 1193 is displayed, the graphical user interface 330 further provides a modification field 1194. The modification field 1194 may be presented after the user selects the modification flag 1193, thereby allowing the user to revise and input at least one correct code name 121 and at least one correct code number 122.

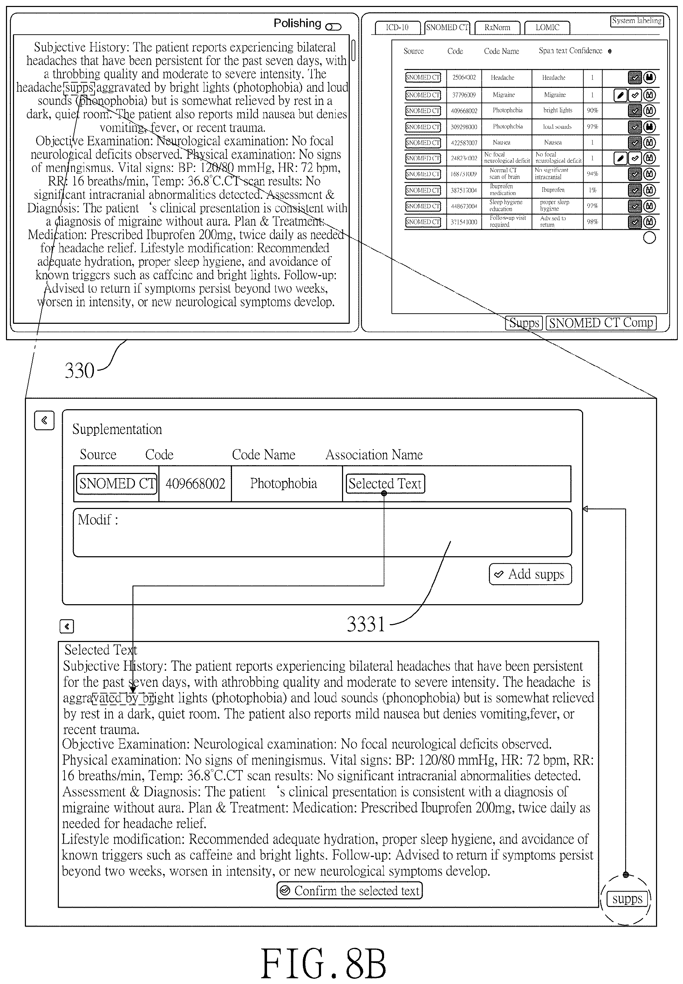

FIG. 8B illustrates a schematic diagram of a supplementation function of the graphical user interface of the electronic medical record standardization system of the present invention. In some embodiments, to address situations in which at least one paragraph 113 or at least one keyword 114 in the unstructured text 112 cannot be recognized and mapped, or in which a user wishes to add remarks to specific content in the unstructured text 112, the graphical user interface 330 may further display a supplementation flag 333 and a supplementation field 3331. The user may select the supplementation flag 333 to present the supplementation field 3331 and enter additional notes or supplemental information therein. In some embodiments, the supplementation flag 333 may be displayed adjacent to selected content after the user selects specific content in the unstructured text 112.

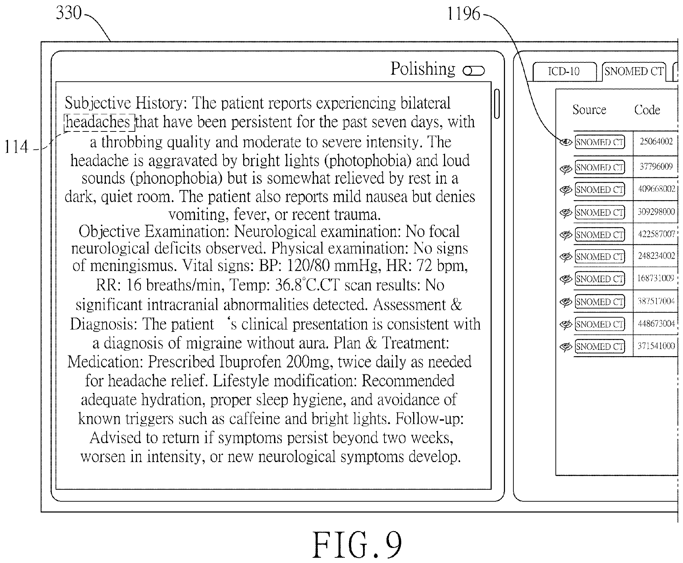

FIG. 9 illustrates a schematic diagram of a focus flag function of the graphical user interface of the electronic medical record standardization system of the present invention. Because an unstructured text 112 may include a large number of mapping entries 119 corresponding to multiple international medical code sets 120, in some embodiments, each mapping entry 119 may further display a focus flag 1196. The user may select the focus flag 1196 such that one or more keywords 114 corresponding to the mapping entry 119 are marked in the unstructured text 112, for example, by bolding, enlarging a font size, underlining, changing a font color, or highlighting.

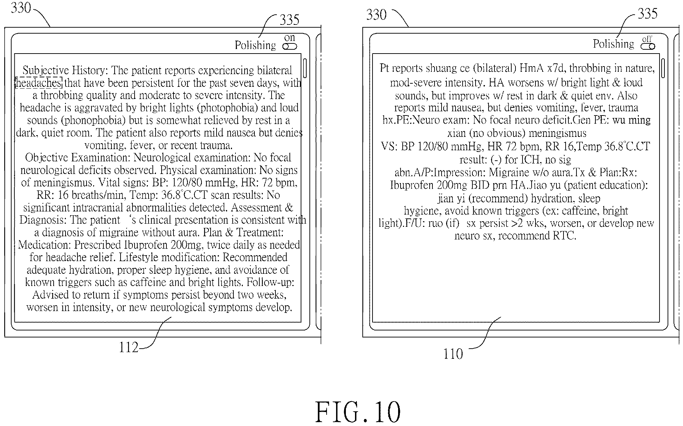

FIG. 10 illustrates a schematic diagram of a refinement-toggle function of the graphical user interface of the electronic medical record standardization system of the present invention. In some embodiments, the graphical user interface 330 further displays a refinement toggle 335, allowing a user to toggle between displaying the unstructured electronic medical record 110 and the unstructured text 112, such that the user can compare differences between the unstructured electronic medical record 110 before algorithmic processing and the unstructured text 112 after algorithmic processing.

In some embodiments, after one or more mapping entries 119 are manually reviewed and confirmed by the user and a standardized electronic medical record 140 is generated, the standardized electronic medical record 140 is transmitted to and stored in the at least one storage device 100. In addition, the standardized electronic medical record 140 may be fed back into the structuring algorithm 230 and the mapping algorithm 240 as training data, thereby continuously improving and optimizing the quality of structuring and mapping.

In some embodiments, the present invention may establish a comprehensive patient timeline record based on structured information in the standardized electronic medical record 140. The timeline may cover care-related information of the patient over a historical period (e.g., the past 10 to 20 years), including visit time, visit location, diagnosis results, chief complaint, test data, prescriptions, follow-up visit frequency, and follow-up status. Such information may be integrated into the graphical user interface 330 such that clinicians and patients can readily understand the patient's history and condition trends.

Through a timeline visualization function, medical and clinical records may be arranged in chronological order, and records from different medical institutions may be distinguished. For example, when the patient has visited multiple hospitals, clinics, and specialty outpatient departments, the system may label an institution name, diagnostic information, and examination results, and may distinguish such records using different colors, markers, or other visual cues. When the patient has multiple follow-up visits, the system may further integrate follow-up intervals, condition changes, and additional test results into the timeline, and indicate trends of improvement, deterioration, or stabilization, thereby enabling physicians to quickly grasp the patient's longitudinal course and formulate treatment plans accordingly.

In addition to individual course tracking, the present invention may also be used for regional healthcare utilization statistics. By analyzing visit locations and diagnosis results in a patient's standardized medical record, the system may generate visit statistics for different counties/cities, hospitals, and clinics, and further analyze regional distributions of medical resources, visit frequency, and service load of medical institutions. In some embodiments, the present invention may further perform disease occurrence trend monitoring. Through standardized patient diagnosis and treatment data, the system may analyze incidence rates of different diseases across different time periods and regions, and monitor potential disease clustering phenomena or epidemic trends.

It should be understood that, without departing from the scope of the present invention, the appended claims encompass both general and specific technical features described herein, and all modifications and equivalent technical solutions consistent with the present invention, regardless of how they are expressed, fall within the scope of protection of the present invention.

## Claims

1. A system for standardizing an electronic medical record, comprising a computer comprising a processor and configured to execute at least three algorithms, wherein the computer is configured to process an unstructured electronic medical record by using the at least three algorithms to generate an unstructured text and at least one mapping entry.

2. The system of claim 1, further comprising a non-standardized medical record stored in the computer.

3. The system of claim 2, wherein the processor is configured to execute a format conversion procedure to process the non-standardized medical record into the unstructured electronic medical record.

4. The system of claim 3, wherein the processor is further configured to execute a format validation procedure to validate the unstructured electronic medical record.

5. The system of claim 1, wherein the at least three algorithms comprise a refinement algorithm configured to perform a readability preprocessing on the unstructured electronic medical record to generate the unstructured text, wherein the unstructured text comprises at least one paragraph.

6. The system of claim 5, wherein the at least three algorithms comprise a structuring algorithm configured to:
extract at least one keyword set from the at least one paragraph;
extract at least one code name set from at least one international medical code set; and
generate a plurality of query sets based on combinations of the at least one keyword set and the at least one code name set.

7. The system of claim 6, wherein the plurality of query sets comprise query sets generated solely from the at least one keyword set and query sets generated solely from the at least one code name set.

8. The system of claim 6, wherein the at least three algorithms further comprise a mapping algorithm configured to:
(a) match a plurality of medical terms comprised in a medical database with the plurality of query sets, wherein each medical term comprises a code combination, and wherein the code combination comprises at least one code name and at least one code number;
(b) for each medical term, obtain a relevance score according to a number of successfully matched query sets, and extract a plurality of top-k medical terms from the plurality of medical terms according to the relevance scores;
(c) perform semantic matching between the plurality of top-k medical terms and the unstructured text to obtain, for each of the plurality of top-k medical terms, a confidence index, and extract at least one target term having a higher confidence index among the plurality of top-k medical terms according to the confidence indices; and
(d) form at least one mapping entry by using the at least one code name and the at least one code number in the code combination comprised in the at least one target term.

9. The system of claim 5, further comprising an electronic device couplable to the computer, wherein the electronic device comprises:
a display device configured to display a graphical user interface (GUI), wherein the GUI is configured to display the unstructured text and the at least one mapping entry; and
a processor coupled to the display device and configured to receive information from the GUI to generate a standardized electronic medical record.

10. The system of claim 9, wherein each mapping entry further comprises the confidence index, a confirmation flag, and a modification flag.

11. The system of claim 9, wherein each mapping entry further comprises:
(i) at least one code name;
(ii) at least one code number;
(iii) at least one keyword;
(iv) a focus flag configured to mark the at least one keyword within the unstructured text; and
(v) an insurance flag.

12. The system of claim 9, wherein the GUI further comprises a modification field and at least one tab configured to categorize the at least one mapping entry from different international medical code sets.

13. The system of claim 9, wherein the GUI further comprises:
(i) a system-flag field comprising at least one flagging strategy;
(ii) a refinement toggle;
(iii) a supplementation flag; and
(iv) a supplementation field.

14. A method for standardizing an electronic medical record, comprising:
Step 1: performing, by a refinement algorithm, a readability preprocessing on an unstructured electronic medical record to generate unstructured text comprising at least one paragraph;
Step 2: performing, by a structuring algorithm, extraction of at least one keyword set from at least one paragraph and extraction of the at least one code name set from at least one international medical code set, and generating a plurality of query sets based on combinations of the at least one keyword set and the at least one code name set;
Step 3: performing, by a mapping algorithm, matching between a plurality of medical terms comprised in a medical database and the plurality of query sets, and extracting at least one mapping entry; and
Step 4: generating a standardized electronic medical record based on mapping between the at least one mapping entry and the unstructured text;
wherein Step 3 further comprises:
Step 3A: matching the plurality of medical terms comprised in the medical database with the plurality of query sets, wherein each medical term comprises a code combination comprising at least one code name and at least one code number;
Step 3B: for each medical term, obtaining a relevance score according to a number of successfully matched query sets, and extracting a plurality of top-k medical terms from the plurality of medical terms according to the relevance scores;
Step 3C: performing semantic matching between the plurality of top-k medical terms and the unstructured text to obtain, for each of the plurality of top-k medical terms, a confidence index, and extracting at least one target term having a higher confidence index among the plurality of top-k medical terms according to the confidence indices; and
Step 3D: forming at least one mapping entry by using at least one code name and at least one code number in the code combination comprised in at least one target term.

15. The method of claim 14, further comprising Step 0: performing a format conversion procedure to process a non-standardized medical record into the unstructured electronic medical record.

16. The method of claim 15, wherein Step 0 further comprises a format validation procedure to validate the unstructured electronic medical record.

17. The method of claim 14, wherein the plurality of query sets comprise query sets generated solely from the at least one keyword set and query sets generated solely from the at least one code name set.

18. The method of claim 14, wherein the confidence index is updatable by repeatedly performing Steps 1 to 3 and updating the confidence index using an algorithm, wherein the algorithm comprises: single-model confidence index, single-model top confidence, single-model multi-run average, multi-model top confidence, multi-model average confidence, and multi-model multi-run average.

19. The method of claim 14, further comprising displaying, via a GUI, the unstructured text and the at least one mapping entry, wherein each mapping entry further comprises the confidence index, a confirmation flag, and a modification flag, and wherein the confirmation flag and the modification flag are displayed based on results calculated by applying a flagging strategy to the confidence index.

20. The method of claim 19, wherein the flagging strategy comprises a threshold configured to evaluate the confidence index of each mapping entry, and wherein the flagging strategy further comprises a weighting setting configured to adjust the confidence index of the at least one mapping entry according to different international medical code sets.

21. The method of claim 19, wherein each mapping entry further comprises:
(i) at least one code name;
(ii) at least one code number;
(iii) at least one keyword;
(iv) a focus flag configured to mark the at least one keyword within the unstructured text; and
(v) an insurance flag.

22. The method of claim 19, wherein the GUI further comprises a modification field, and at least one tab configured to categorize the at least one mapping entry from different international medical code sets.

23. The method of claim 19, wherein the GUI further comprises:
(i) a system-flag field comprising at least one flagging strategy;
(ii) a refinement toggle; and
(iii) a supplementation flag and a supplementation field.

24. The method of claim 19, wherein the standardized electronic medical record is usable to train and optimize the structuring algorithm and the mapping algorithm.

25. The method of claim 19, wherein the GUI is further used for patient-course visualization and medical statistical analysis, comprising:
(i) establishing and displaying, on the GUI, a patient course timeline based on visit time, visit location, diagnosis results, chief complaint, test data, prescriptions, follow-up visit count, and follow-up status in the standardized electronic medical record;
(ii) generating regional visit statistics based on visit locations and diagnosis results in the standardized electronic medical record to analyze visit frequency across cities/counties, hospitals, and clinics and to evaluate medical resource distribution and service load, and displaying the regional visit statistics on the GUI; and
(iii) monitoring disease occurrence trends by analyzing the at least one mapping entry in the standardized electronic medical record to evaluate incidence rates of a specific disease across different regions and time periods, and displaying disease clustering or epidemic trends on the GUI.
